# EUROPEAN PATENT APPLICATION

(11) **EP 1 046 715 A1**
(43) Date of publication of application: **25.10.2000**
(21) Application number: 99108074.8
(22) Date of filing: 23.04.1999
(51) Int. Cl.: C12Q 1/42, A61K 38/46, A61K 48/00

(54) **Interaction of vascular-endothelial protein-tyrosine phosphatase with the angiopoietin receptor tie-2**

(71) Applicant: Max-Planck-Gesellschaft zur Förderung der Wissenschaften e.V., 80539 München (DE)
(72) Inventor: Fachinger, Gregor, 10551 Berlin (DE); Risau, Barbara, 35510 Butzbach (DE); Deutsch, Urban Dr., 61231 Bad Nauheim (DE)
(74) Representative: Weiss, Wolfgang, Dipl.-Chem. Dr.

(57) **Abstract**

The present invention relates to a method for monitoring or modulating the activity of the angiopoietin receptor-type tyrosine kinase Tie-2.

## Description

The present invention relates to a method for monitoring or modulating the activity of the angiopoietin receptor-type tyrosine kinase Tie-2.

A key mechanism in the proliferation and differentiation control of all cells are membrane-located receptors, whose activation in many cases is mediated by external factors via phosphorylation of tyrosine residues. The mutation of a series of endothelial cell specific receptor-tyrosine kinases (RTKs) results in lethal phenotypes early during murine embryonal development (Hanahan, Science 277 (1997), 48 - 50; Risau, Nature 386 (1997), 671 - 674). The proliferation and differentiation of endothelial cells depends on two receptor tyrosine kinase systems. The vascular endothelial growth factor (VEGF) is a secreted angiogenic factor and promotes vascularization by activation of its high affinity receptors VEGFR-1 (FIt-1) or VEGFR-2 (Flk-1). The RTKs Tie-1 and Tie-2 are involved in the sprouting and remodelling of the embryonic vascular system. The activity of these kinases is regulated by the recently identified ligands, the angiopoietins.

After ligand binding RTKs are activated by phosphorylation on tyrosine residues. Specific protein-tyrosine phosphatases (PTPs) are involved in the fine-tuning of RTK activity. Several classes of PTPs have been identified. However, the biological functions thereof are presently not understood (Neel & Tonks, Curr. Opin. Cell Biol. 9(1997), 193 - 204; Streuli, Curr. Opin. Cell Biol. 8 (1996), 182 - 188).

In a study to identify PTPs in endothelial cells a murine vascular-endothelial protein-tyrosine phosphatase VE-PTP was identified (VE-PTP: a receptor protein-tyrosine phosphatase expressed in vascular endothelium, EMBO-FEBS Workshop on Protein Phosphatases and Protein Dephosphorylation, Oxford, UK, September 21 - 26, 1997). Indications for a functional interaction between VE-PTP and a receptor-type kinase have not been described, however. Further, the association of PTPs with their substrates is difficult to determine due to the transcient nature of the enzyme substrate association (Flint et al., Proc. Natl. Acad. Sci. U.S.A. 94 (1997), 1680 - 1685).

The experiments underlying the present application discovered that VE-PTP is a homolog of the human HPTPβ (Krueger et al., EMBO J., 9, (1990), 3241 - 3252), and that it is specifically expressed in endothelial cells both during the embryonal development of mice and in brain capillary vessels of newborn animals. Biochemical analyses using VE-PTP trapping mutants show a specific interaction between the C-terminal part of the molecule which contains the catalytic domain and the RTK Tie-2 but not with the vascular endothelial growth factor receptor VEGFR-2. Moreover, a dephosphorylation of Tie-2 could be detected in the presence of VE-PTP in transiently transfected COS-1 cells. These data identify Tie-2 as a specific substrate for VE-PTP and show that it is a specific modulator of Tie-2 activity.

This result is of high clinical relevance, as Tie-2 holds a key position in angiogenetic processes, the formation of the blood vessel system during embryonal development, the healing of wounds as well as in pathological processes, e.g. tumor development. As VE-PTP shows a specific interaction with Tie-2 and can modulate the tyrosine phosphorylation of the latter, the receptor-protein tyrosine phosphatase is a target both for diagnostic monitoring and for therapeutically influencing the said processes.

Thus, a subject matter of the present invention is the use of vertebrate, e.g. mammalian vascular-endothelial protein-tyrosine phosphatases or portions thereof for the manufacture of an agent for monitoring or modulating the activity of receptor-type tyrosine kinase Tie-2.

A further subject matter of the present invention is the use of nucleic acids encoding vertebrate, e.g. mammalian vascular-endothelial protein-tyrosine phosphatases or portions thereof for the manufacture of an agent for monitoring or modulating the activity of receptor-type tyrosine kinase Tie-2.

Still a further subject matter of the invention is the use of ligands for vertebrate, e.g. mammalian vascular-endothelial protein-tyrosine phosphatases for the manufacture of an agent for monitoring or modulating the activity of receptor-type tyrosine kinase Tie-2.

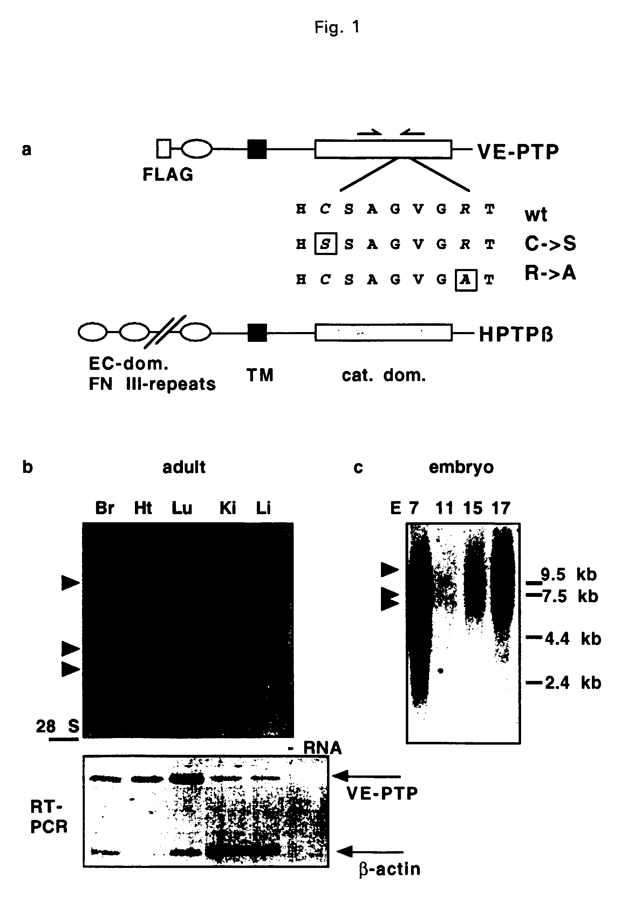

The vascular-endothelial protein-tyrosine phosphatases and nucleic acids coding therefor, e.g. genes or cDNA molecules, are obtainable from vertebrate cells, preferably from mammalian endothelial cells, e.g. murine or human cells. Preferably the vascular-endothelial protein-tyrosine phosphatase is selected from murine phosphatase VE-PTP, human phosphatase HPTPβ or portions thereof, particularly portions comprising the catalytic domain which is located at the C-terminus of the molecule (Fig. 1 a). The nucleic acid sequence and the corresponding amino acid sequence of murine vascular-endothelial protein-tyrosine phosphatase are depicted in SEQ. ID. NO 1 and 2, respectively. The corresponding sequences of the human protein, which were identified by Krueger et al. (supra) are depicted in SEQ. ID. NO 3 and 4.

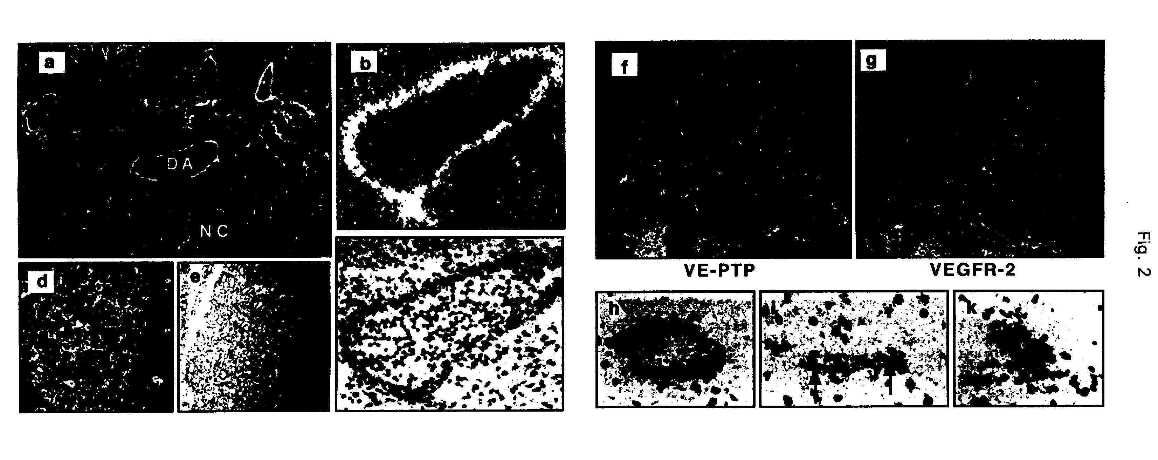

The polypeptide or a portion thereof is suitable for monitoring or modulating the activity of receptor-type tyrosine kinase Tie-2. In addition to a phosphatase with unmodified sequence of the catalytic domain also mutants thereof, which show a modified, e.g. enhanced binding to Tie-2, e.g. the trapping mutants as depicted in Fig. 2 are suitable for the present invention. Particularly mutants, which exhibit an enhanced binding to Tie-2 are well suited for diagnostic and therapeutic applications.

The interaction between the vascular endothelial protein-tyrosine phosphatase and its substrate Tie-2 can also be monitored and/or modulated on the nucleic acid level. To this end nucleic acids, e.g. DNA molecules, RNA molecules or artificial nucleic acid analogs such as peptidic nucleic acids may be used. Preferably these nucleic acids comprise at least 15, particularly at least 20 nucleotides from murine phosphatase VE-PTP gene, human phosphatase HPTPβ gene or sequences complementary thereto. These nucleic acids are suitable for the determination of the PTP expression by using known hybridization or/and amplification techniques such as PCR. On the other hand, nucleic acids can be used for the modulation of the VE-PTP expression in the form of antisense constructs or as ribozymes.

A still further aspect of the invention is the use of ligands for vertebrate, e.g. mammalian vascular endothelial-protein tyrosine phosphatases. Examples of such ligands are antibodies, e.g. polyclonal or monoclonal antibodies and antibody fragments. Polyclonal antibodies are available according to known protocols by immunization of test animals with purified VE-PTP, HPTPβ or partial fragments thereof, which preferably contain the catalytic domain. From these test animals monoclonal antibodies can be generated in a known manner by using the method applied by Koehler and Milstein. The polyclonal or monoclonal antibodies can also be used in the form of fragments which are obtainable by proteolyic treatment or genetic engineering.

One embodiment of the invention concerns the monitoring or detection of the Tie-2 activity. This detection can be carried out by using known methods, e.g. using labelled polypeptides, nucleic acids or antibodies. A further embodiment concerns the modulation of the Tie-2 activity. Thereby a stimulation or a repression of the Tie-2 activity is possible.

Of major importance is the examination or influencing of the interaction between VE-PTP and Tie-2 for angiogenesis. Thus the present invention provides means for inducing or for inhibiting vascular growth or remodelling and blood vessel maturation. Particularly, the present invention provides means for inhibiting tumor growth and formation of tumor metastases, e.g. by repressing Tie-2 activity in target cells.

Moreover, the invention is explained by the following figures and sequence protocols.
- Fig. 1a: shows the schematic representation of VE-PTP, its genetically engineered trapping mutants and HPTPβ.
- Fig. 1b and c: show Northern blot and RT-PCR analyses of VE-PTP expression in mouse tissues and during mouse embryonic development.
- Fig. 2: shows *in vivo* expression analysis of VE-PTP by *in situ* hybridization.
- Fig. 3: shows biochemical interactions of VE-PTP trapping mutants with Tie-2 protein.
- Fig. 4: shows selective dephosphorylation of Tie-2, but not VEGFR-2 by wild-type VE-PTP.

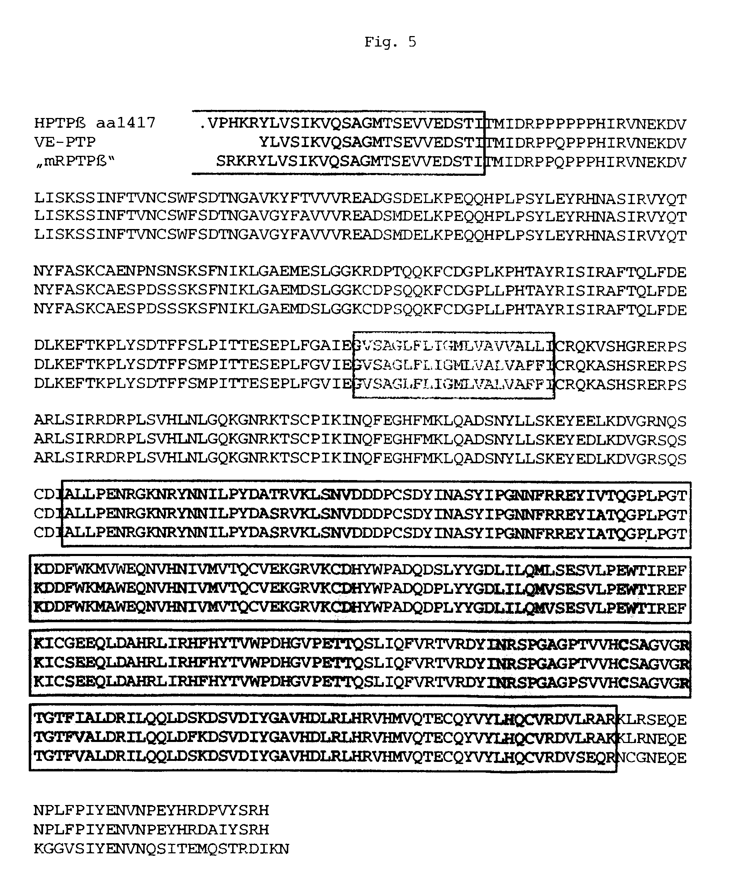

- Fig. 5: shows a sequence comparison of the C-terminus of HPTPβ with VE-PTP and the translated "mRPTPβ" sequence. Known protein domains are depicted: Membrane proximal FN III-domain (blue), transmembrane domain (red) and catalytic domain (green). The catalytic center is characterized by a C(x)₅R-motif.
- SEQ. ID. NO. 1 and 2: show the nucleotide sequence of VE-PTP cDNA and the corresponding amino acid sequence.
- SEQ. ID. NO. 3 and 4: show the nucleotide sequence of HPTPβ cDNA and the corresponding amino acid sequence.

### Example 1

A PCR screen of a murine brain capillary cDNA library and reverse transcribed mRNA of bEND5 endothelioma cells to identify endothelial specific members of the protein-tyrosine phosphatase family was performed. For PCR, 100 pmol degenerated primers RPTP1 5'-GA(C/T) TT(C/T) TGG ATG (A/G/T) (G/T)I TGG GA-3' and RPTP2 5'-CCI ACI CGI GCI (G/C)(A/T)(A/G) CA(A/G) TGI AC-3' in 50 µl reactions were used. As templates 1.25 µg λ-DNA from mouse P4-10 brain capillary-library (Schnürch & Risau, Development, 119 (1993), 957 - 968) or 3 µl of Superscript cDNA preparation (GIBCO BRL) from bEND5 mRNA were used. Isolated 370 bp products were cloned into the vector pCRII (Invitrogen), analysed by restriction cleavage and sequenced on an ABI 370 automated sequencer (Applied Biosystems).

One of the identified PCR products encodes a polypeptide, designated as vascular-endothelial protein-tyrosine phosphatase (VE-PTP) which was identified as murine homolog of the previously described receptor-type protein-tyrosine phosphatase HPTPβ (Krueger et al. EMBO J. 9 (1990), 3241 - 3252). VE-PTP and HPTPβ belong to the subclass III of receptor-type PTPs bearing exclusively fibronectin type III-like repeats in the extracellular domain and a single catalytic domain in the cytoplasmatic tail (Fig. 1a) (Brady-Kalnay & Tonks, Curr. Opin. Cell. Biol. 7 (1995), 650 - 657).

Fig. 1a shows a schematic representation of VE-PTP, its genetically engineered trapping mutants C- > S, R- > A and HPTPβ. Rectangles indicate mutated amino acids in the catalytic core. The location of the degenerated primers used in the PCR screen are indicated by arrows (EC-dom., extracellular domain; FN III fibronectin-type III-like repeat; cat. dom., catalytic domain).

### Example 2

A Northern blot and RT-PCR analysis of VE-PTP expression in mouse tissues and during mouse embryonic development were performed. A 751 bp EcoRI-fragment from VE-PTP part 1, obtained by PCR using primers PrPTPβfor 5'-GGA AGA GGT ACC TGG TGT CCA TCA AGG-3' and PrPTPβrev 5'-GGC CGG TCC CTA CGA ATG CTG AGC CGG GCA G-3' deduced from a partial clone of murine "RPTPβ" (Schepens et al. Mol. Biol. Reports, 16 (1992)), and cloned in the vector pBS KS( +)(Stratagene), was labelled with α³²P-dCTP (Amersham Pharmacia Biotech). For Northern blot analysis 20 µg of total RNA from mouse tissues (Chomczynski & Sacchi, Analyt. Biochem. 162 (1987), 156 - 159) were loaded on a formaldehyde containing agarose gel and blotted. A mouse embryo mRNA Northern blot was obtained from Clontech and hybridization was carried out according to manufacturer's instructions. Autoradiography was performed at -70° C for 17 d. For semiquantitative PCR 50 µl reactions containing 2 µl of reverse transcribed cDNA preparations and 20 pmol of primers betaseq2 5'- CCC TCT CCC TTC CTA CCT GG-3' and betarev 5'- GGC CGG TCC CTA CGA ATG CTG AGC CGG GCA GG-3' were used, giving a 416 bp fragment. 30 cycles PCR was optimized to detect 1 fg of VE-PTP plasmid DNA. β-actin RT-PCR was performed as described (Nakajima-lijima et al, Proc. Natl. Acad. Sci. U.S.A. 82 (1985), 6133 - 6137).

Northern blot analysis of VE-PTP expression revealed a major transcript of approximately 11 kB and two additional transcripts of 7.5 and 6 kB. In the adult mouse VE-PTP mRNA was strongly expressed in brain as well as in lung and heart. Very weak expression was detectable in kidney and liver (Fig. 1 b). These data were confirmed by semi-quantitative RT-PCR performed with RNA from these organs (Fig. 1b). During embryonic development VE-PTP was weakly expressed at embryonic day E11, expression increased at E15 reaching a maximum at E17 (Fig. 1c). Strong expression was detected at E7, which may result from expression in contaminating maternal tissue as expression in the placenta was observed by *in situ* hybridization analysis as well.

### Example 3

An *in vivo* expression analysis of VE-PTP by *in situ* hybridization to frozen sections of mouse embryonic tissues was carried out. The results are shown in Fig. 2. Fig. 2a is a darkfield image of an E12.5 embryo section hybridized with a VE-PTP antisense probe. (NC: neural crest, DA: dorsal aorta). Fig. 2b is a darkfield image and Fig. 2c is a brightfield image of a higher magnification of the vessel indicated in a (asteriks). Fig. 2d - h are sagittal sections of E15.5 embryos hybridized with antisense VE-PTP probes. Fig. 2d is a darkfield image and Fig. 2e a brightfield image of the lung. Fig. 2f is a darkfield image of the head region. Fig. 2g is an E15.5 embryo section hybridized with a VEGFR-2 antisense probe. Fig. 2h - k are vessels in brain sections of P10 mice hybridized with antisense VE-PTP probes. As templates for *in vitro* transcription pCRII (Invitrogen) VE-PTP-1 (370 bp fragment of VE-PTP coding for protein sequence corresponding to aa 1786 - 1913 in HPTPβ in pCRII) and pBS VE-PTPpart1 were used. Sectioning of mouse embryos and *in situ* hybridization were performed as described (Breier et al, Development, 114 (1992), 521 - 532).

At the earliest timepoint analysed (E9.5), expression was detectable in the endothelial cell layer lining the dorsal aortae. During the subsequent developmental stages VE-PTP expression was increased throughout the developing vascular system (Fig. 2a). Strong hybridization signals were visible in endothelial cells forming blood vessels, whereas no specific signals were detected in blood cells or smooth muscle cells surrounding the vessels (Fig. 2b, c). At E15.5 specific signals were detectable in all organs with highest expression in the lung (Fig. 2d,e). Comparison to serial sections hybridized with an antisense probe to VEGFR-2 (Flk-1) as an endothelial cell marker, confirmed the vascular endothelial specific expression pattern of VE-PTP (Fig. 2 f,g). In contrast to the uniform expression levels of VEGFR-2 in different types of embryonic endothelial cells, VE-PTP was more strongly expressed in endothelial cells lining larger, smooth muscle cell invested vessels than those of small capillaries and veins. On brain sections of newborn mice, specific expression of VE-PTP was detectable in brain capillaries as well as in larger vessels (Fig. 2h-k). No specific signals were visible in neuronal or glial cells.

### Example 4

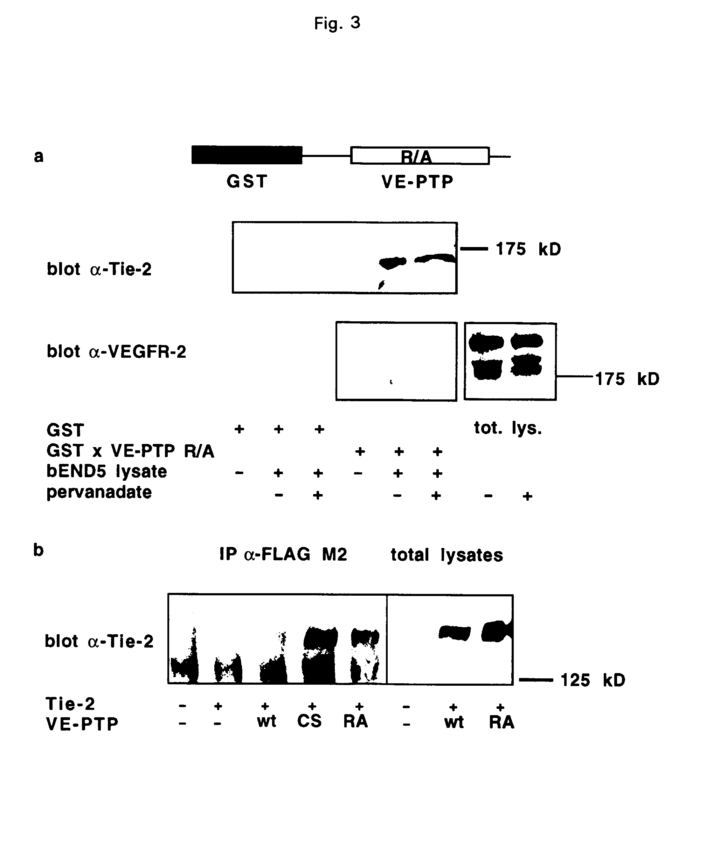

The biochemical interactions of VE-PTP with the receptor tyrosine kinases Tie-2 and VEGFR-2 were investigated using bacterial GST-fusion proteins. The results are shown in Fig. 3.

Fig. 3a demonstrates the results of GST-fusion pull down experiments. GST and GST x VE-PTP R/A fusion protein were incubated with lysates from bEND5 cells. Precipitates were blotted with an anti-Tie-2 antibody and reblotted with an VEGFR-2 specific antibody. (tot. lys.: total lysates of bEND5 cells). pGEX-VE-PTP contains a 1.1 kB 3' part of EST-clone 552065 (Lennon et al., Genomics 33 (1996), 151 -152) coding for the cytoplasmic domain of VE-PTP cloned in pGEX 3T (Amersham Pharmacia Biotech). GST and GST-fusion proteins were expressed in *E.coli strain* TOP10 essentially as described (Frangioni & Neel, Anal. Biochem. 210 (1993), 179 - 187). For pull down experiments 10 cm dishes of confluent endothelial cells were pretreated with pervanadate, lysed and incubated with 10 µg of GST-fusion protein prebound to glutathion-sepharose as described before (Jallal et al., J. Biol. Chem. 272 (1997), 12158 - 12163).

Fig. 3b shows co-immunoprecipitation of VE-PTP trapping mutants (C- > S, R- > A) with Tie-2. COS⁻¹ cells were transfected with FLAG-tagged VE-PTP and trapping mutants together with Tie-2. Immunoprecipitation was performed with anti-FLAG antibody M2. Precipitates were blotted with a Tie-2 specific monoclonal antibody.

pCMV-FLAG VE-PTP wt, C- > S and R- > A contain cDNA sequences coding for a polypeptide stretch corresponding to aa 1418-1977 in HPTPβ cloned in pCMV-FLAG-1 (Kodak). Trapping mutations C->S and R->A were introduced by PCR mutagenesis using primer Prbetamutcs 5'-TCC GTA GTG CAC TCG AGT GCT GGT GTG-3' and primer Prbetamutra 5'-GCT GGT GTG GGC GCC ACA GGG ACG TTC-3'. COS-1 cells (Gluzman, Cell 23 (1981), 175 - 182) were transfected using the modified calcium phosphate method (Chen & Okayama, Mol. Cell. Biol. 7 (1987), 2745 - 2752). For transfection 10 µg of pCMV-FLAG derivates and 2 µg of expression plasmids coding for the RTKs were used. As control 0.5 µg of EGFP expression plasmid (Clontech) were cotransfected. Cells were harvested after 2 d of expression. Transfection efficiency was evaluated under fluorescent light and was usually between 30 - 70%.

In mixing experiments of endothelial cell lysates and trapping mutants of the VE-PTP catalytic domain fused to GST, we detected interaction with the Tie-2 receptor, while GST alone did not precipitate Tie-2. The interaction was independent of pretreatment with pervanadate. In these assays coprecipitation of VEGFR-2 was never detectable (Fig. 3a).

To test for potential substrate interactions with Tie-2 and VEGFR-2 we coexpressed these RTKs with either a FLAG-tagged version of VE-PTP corresponding to aa 1418-1997 of HPTPβ, or the respective trapping mutants (Fig. 1a). Physical association was analysed by co-immunoprecipitation using an anti-FLAG-antibody and subsequent blotting of the precipitates with antibodies specific for the repective RTK. In this assay the Tie-2 receptor co-precipitated with both trapping mutants of VE-PTP (C- > S, R- > A) (Fig. 3b). The wild type phosphatase failed to precipitate Tie-2 efficiently, even though the receptor was expressed at comparable levels. This reduced association of PTPs *in vitro* with their substrates is due to the transient nature of the enzyme substrate association. Unlike Tie-2, VEGFR-2 could neither be co-immunoprecipitated with VE-PTP nor with one of the trapping mutants, even though VEGFR-2 expression was comparable to that of Tie-2.

### Example 5

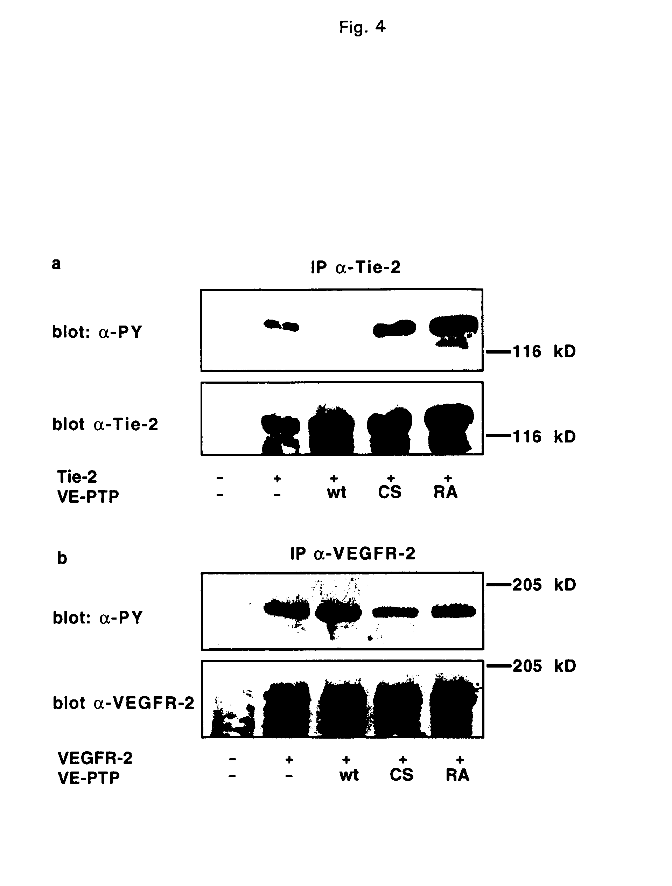

Finally, the phosphorylation state of RTKs was determined in the presence of VE-PTP. Figure 4 shows dephosphorylation of (a) Tie-2 but not (b) VEGR-2 by wild-type VE-PTP. RTKs were immunoprecipitated with specific antibodies from cotransfected COS-1 cells. Precipitates were blotted with anti-phosphotyrosine antibodies and after stripping reprobed with RTK-specific antibodies.

Tie-2 and VEGFR-2 expression vectors were published previously (Koblizek et al., Curr. Biol. 8 (1997), 529 - 532; Millauer et al., Cell 72 (1993), 835 - 846). Rat monoclonal antibodies against Tie-2 clones 3g1 and 4g8 (Koblizek et al. (1997) supra) and Flk-1 clone 12α1 (Kataoka et al., Devel. Growth Diff. 39 (1997), 729 - 740) were used. Immunoprecipitations were performed with 5 µg of the monoclonal antibodies and immunoblotting with 2 µg/ml. Polyclonal anti-Flk-1 serum 1D3 (Sugen) was used in a 1:5000 dilution. Monoclonal anti-Flag antibody M2 (Kodak), polyclonal antiserum against Tie-2 (Santa Cruz Biotechnology) and monoclonal mouse antibody against phosphotyrosine PY20 (Transduction Labs) were used according to the manufacturer's instructions. Immunoprecipitations and immunoblotting were performed as described before (Esser et al., J. Cell. Biol. 140(1998), 947 - 959); Jallal et al., J. Cell. Biol. Chem. 272 (1997), 12158 - 12162).

Immunoprecipitates of VEGFR-2 and Tie-2 co-expressed with either the VE-PTP trapping mutants (C- > S, R- > A) or wt VE-PTP were blotted with an α-phosphotyrosine-specific antibody and then reprobed with antibody specific for the RTK. Only for Tie-2, changes in the phosporylation status were observed. In the presence of the trapping mutants (C->S, R->A) the receptor was reproducibly more highly phosphorylated than in the controls. This hyperphosphorylation of Tie-2 in the presence of catalytically impaired trapping mutants suggests that physical interaction leads to protection of the receptor from dephosphorylation. In contrast, hypophosphorylation of the Tie-2 receptor was observed in the presence of wt VE-PTP, when compared to vector control (Fig. 4a). No significant changes were detected in the phosphorylation status of VEGFR-2, irrespective of the presence of VE-PTP or its trapping mutants (Fig. 4b). These findings clearly show that Tie-2 is a specific substrate for the endothelial specific phosphatase VE-PTP.

## Claims

1. Use of vertebrate vascular-endothelial protein-tyrosine phosphatases or portions thereof for the manufacture of an agent for monitoring or modulating the activity of receptor-type tyrosine kinase Tie-2.

2. The use of claim 1 wherein said phosphatase is selected from murine phosphatase VE-PTP, human phosphatase HPTPβ or portions thereof.

3. The use of claim 1 or 2 wherein said portion comprises the catalytic domain.

4. Use of nucleic acids encoding vertebrate vascular-endothelial protein-tyrosine phosphatases or portions thereof for the manufacture of an agent for monitoring or modulating the activity of receptor-type tyrosine kinase Tie-2.

5. The use of claim 4 wherein said nucleic acid comprises at least 15 nucleotides from murine phosphatase VE-PTP nucleic acid, human phosphatase HPTPβ nucleic acid or sequences complementary thereto.

6. The use of ligands for vertebrate vascular-endothelial protein-tyrosine phosphatases for the manufacture of an agent for monitoring or modulating the activity of receptor-type tyrosine kinase Tie-2.

7. The use of claim 7 wherein said ligands are selected from antibodies and antibody fragments.

8. The use of any one of claims 1 - 7 for detecting Tie-2 activity.

9. The use of any one of claims 1 - 7 for stimulating Tie-2 activity.

10. The use of any one of claims 1 - 7 for repressing Tie-2 activity.

11. The use of any one of the previous claims for monitoring or modulating angiogenesis.

12. The use of any one of the previous claims for inducing vascular growth or remodelling and blood vessel maturation.

13. The use of any one of the previous claims for inhibiting vascular growth or remodelling and blood vessel maturation.

14. The use of any one of the previous claims for inhibiting tumor growth.

15. The use of any one of the previous claims for inhibiting formation of tumor metastases.


### Annex to the application documents - subsequently filed sequences listing

SEQUENCE LISTING

<110> Max-Planck-Gesellschaft zur Förderung der Wissensc

<120> Interaction of vascular-endothelial protein-tyrosine
      phosphatase with the Angiopoietin receptor Tie-2

<130> 20036PEP

<140>
<141>

<160> 4

<170> PatentIn Ver. 2.1

<210> 1
<211> 1839
<212> DNA
<213> mouse

<220>
<221> CDS
<222> (1)..(1740)

<400> 1

aag agg tac ctg gtg tcc atc aag gtg cag tcg gcc ggc atg acc agt    48

Lys Arg Tyr Leu Val Ser Ile Lys Val Gln Ser Ala Gly Met Thr Ser
  1               5                  10                  15

gag gtg gtt gaa gat agc acc atc acc atg ata gac cgc ccg cct caa    96

Glu Val Val Glu Asp Ser Thr Ile Thr Met Ile Asp Arg Pro Pro Gln
             20                  25                  30

ccg cct cca cac atc cgt gtg aat gaa aag gat gtg cta atc agc aaa   144

Pro Pro Pro His Ile Arg Val Asn Glu Lys Asp Val Leu Ile Ser Lys
         35                  40                  45

tct tcc atc aac ttt act gtc aac tgc agc tgg ttc agc gac acc aac   192

Ser Ser Ile Asn Phe Thr Val Asn Cys Ser Trp Phe Ser Asp Thr Asn
     50                  55                  60

gga gcg gtt ggg tac ttt gct gtg gtg gtg aga gag gcc gac agc atg   240

Gly Ala Val Gly Tyr Phe Ala Val Val Val Arg Glu Ala Asp Ser Met
 65                  70                  75                  80

gat gag ttg aag cca gaa cag cag cac cct ctc cct tcc tac ctg gag   288

Asp Glu Leu Lys Pro Glu Gln Gln His Pro Leu Pro Ser Tyr Leu Glu
                 85                  90                  95

tac aga cac aac gcc tcc atc cga gtc tac cag acc aat tat ttt gcc   336

Tyr Arg His Asn Ala Ser Ile Arg Val Tyr Gln Thr Asn Tyr Phe Ala
            100                 105                 110

agc aaa tgt gct gaa agt ccc gac agc agt tct aaa agt ttc aac att      384

Ser Lys Cys Ala Glu Ser Pro Asp Ser Ser Ser Lys Ser Phe Asn Ile
        115                 120                 125

aag ctt gga gca gag atg gac agc ctc ggt ggc aaa tgt gat ccc agt      432

Lys Leu Gly Ala Glu Met Asp Ser Leu Gly Gly Lys Cys Asp Pro Ser
    130                 135                 140

cag cag aaa ttc tgt gat gga ccg ctg ttg cca cac acc gcc tac aga      480

Gln Gln Lys Phe Cys Asp Gly Pro Leu Leu Pro His Thr Ala Tyr Arg
145                 150                 155                 160

atc agc atc cgg gct ttt aca cag cta ttt gac gag gac ttg aaa gag      528

Ile Ser Ile Arg Ala Phe Thr Gln Leu Phe Asp Glu Asp Leu Lys Glu
                165                 170                 175

ttc acc aaa cct ctc tac tcg gat acg ttc ttc tct atg ccc atc acc      576

Phe Thr Lys Pro Leu Tyr Ser Asp Thr Phe Phe Ser Met Pro Ile Thr
            180                 185                 190

aca gag tca gag ccc ttg ttt gga gtt att gaa ggt gtg agt gct ggc      624

Thr Glu Ser Glu Pro Leu Phe Gly Val Ile Glu Gly Val Ser Ala Gly
        195                 200                 205

ctg ttt cta att ggc atg ctg gtg gcc ctt gtt gcc ttc ttc atc tgc      672

Leu Phe Leu Ile Gly Met Leu Val Ala Leu Val Ala Phe Phe Ile Cys
    210                 215                 220

aga cag aaa gct agc cac agc agg gaa agg cca tct gcc cgg ctc agc      720

Arg Gln Lys Ala Ser His Ser Arg Glu Arg Pro Ser Ala Arg Leu Ser
225                 230                 235                 240

att cgt agg gac cgg cct ttg tct gtc cat ctg aat ctg ggc cag aaa      768

Ile Arg Arg Asp Arg Pro Leu Ser Val His Leu Asn Leu Gly Gln Lys
                245                 250                 255

ggc aac cgg aaa act tct tgc ccc ata aag atc aat cag ttt gaa ggg      816

Gly Asn Arg Lys Thr Ser Cys Pro Ile Lys Ile Asn Gln Phe Glu Gly
            260                 265                 270

cat ttc atg aag ctg cag gca gac tcc aac tac ctt cta tcc aag gaa      864

His Phe Met Lys Leu Gln Ala Asp Ser Asn Tyr Leu Leu Ser Lys Glu
        275                 280                 285

tat gag gac tta aaa gac gtg ggt aga agc cag tca tgt gac att gcc      912

Tyr Glu Asp Leu Lys Asp Val Gly Arg Ser Gln Ser Cys Asp Ile Ala
    290                 295                 300

ctc ttg cct gag aat cga ggg aaa aat cga tac aac aac ata ttg cct      960

Leu Leu Pro Glu Asn Arg Gly Lys Asn Arg Tyr Asn Asn Ile Leu Pro
305                 310                 315                 320

tat gat gcc tca aga gtg aag ctc tcg aat gtc gat gac gac cct tgc 1008

Tyr Asp Ala Ser Arg Val Lys Leu Ser Asn Val Asp Asp Asp Pro Cys
                325                 330                 335

tct gac tac atc aac gcc agc tac atc ccc ggt aac aac ttc aga cga 1056

Ser Asp Tyr Ile Asn Ala Ser Tyr Ile Pro Gly Asn Asn Phe Arg Arg
            340                 345                 350

gaa tac atc gcc act cag gga ccg ctt cca ggc acc aag gat gac ttc 1104

Glu Tyr Ile Ala Thr Gln Gly Pro Leu Pro Gly Thr Lys Asp Asp Phe
        355                 360                 365

tgg aag atg gcg tgg gag cag aac gtt cac aac atc gtc atg gtg acc 1152

Trp Lys Met Ala Trp Glu Gln Asn Val His Asn Ile Val Met Val Thr
    370                 375                 380

cag tgt gtt gaa aag ggc cga gtg aag tgt gac cat tac tgg cca gca 1200

Gln Cys Val Glu Lys Gly Arg Val Lys Cys Asp His Tyr Trp Pro Ala
385                 390                 395                 400

gac cag gac ccc ctc tac tac ggt gat ctc atc cta cag atg gtc tcg 1248

Asp Gln Asp Pro Leu Tyr Tyr Gly Asp Leu Ile Leu Gln Met Val Ser
                405                 410                 415

gag tcc gtg ctc ccc gag tgg acc atc agg gag ttt aag ata tgc agt 1296

Glu Ser Val Leu Pro Glu Trp Thr Ile Arg Glu Phe Lys Ile Cys Ser
            420                 425                 430

gaa gaa cag ttg gat gca cac aga ctc atc cgt cac ttt cac tac acg 1344

Glu Glu Gln Leu Asp Ala His Arg Leu Ile Arg His Phe His Tyr Thr
        435                 440                 445

gtg tgg cca gac cat ggg gtc cca gag acc acc cag tcc ctg atc caa 1392

Val Trp Pro Asp His Gly Val Pro Glu Thr Thr Gln Ser Leu Ile Gln
    450                 455                 460

ttt gtg agg aca gtc agg gac tac atc aac aga agc ccc ggg gct ggg 1440

Phe Val Arg Thr Val Arg Asp Tyr Ile Asn Arg Ser Pro Gly Ala Gly
465                 470                 475                 480

ccc acc gta gtg cac tgc agc gct ggt gtg ggc aga aca ggg acg ttc 1488

Pro Thr Val Val His Cys Ser Ala Gly Val Gly Arg Thr Gly Thr Phe
                485                 490                 495

gtt gcc ctg gac cgg atc ctc cag cag ttg gac ttt aag gac tcc gtg 1536

Val Ala Leu Asp Arg Ile Leu Gln Gln Leu Asp Phe Lys Asp Ser Val
            500                 505                 510

gac att tat ggg gca gtg cat gac cta aga ctc cac agg gtt cac atg 1584

Asp Ile Tyr Gly Ala Val His Asp Leu Arg Leu His Arg Val His Met
        515                 520                 525

gtc cag acc gag tgt caa tat gtg tat ctg cat cag tgt gta aga gac 1632

Val Gln Thr Glu Cys Gln Tyr Val Tyr Leu His Gln Cys Val Arg Asp
    530                 535                 540

gtc ctc aga gca aag aaa ctg cgg aac gag caa gag aac ccc ctg ttt 1680

Val Leu Arg Ala Lys Lys Leu Arg Asn Glu Gln Glu Asn Pro Leu Phe
545                 550                 555                 560

ccg att tat gag aat gtg aat cca gag tat cac aga gat gca atc tac 1728

Pro Ile Tyr Glu Asn Val Asn Pro Glu Tyr His Arg Asp Ala Ile Tyr
                565                 570                 575

tcg aga cat taa gaattcacct gaagatcccc tggataaaag cgtttcactg 1780

Ser Arg His
580

tgtgacttta aaaaaaaaaa aaaaaaaaac tcgagggggg gcccgtaccc aatcnnaaa 1839

<210> 2
<211> 580
<212> PRT
<213> mouse

<400> 2
Lys Arg Tyr Leu Val Ser Ile Lys Val Gln Ser Ala Gly Met Thr Ser
1               5                   10                  15

Glu Val Val Glu Asp Ser Thr Ile Thr Met Ile Asp Arg Pro Pro Gln
            20                  25                  30

Pro Pro Pro His Ile Arg Val Asn Glu Lys Asp Val Leu Ile Ser Lys
        35                  40                  45

Ser Ser Ile Asn Phe Thr Val Asn Cys Ser Trp Phe Ser Asp Thr Asn
    50                  55                  60

Gly Ala Val Gly Tyr Phe Ala Val Val Val Arg Glu Ala Asp Ser Met
65                  70                  75                  80

Asp Glu Leu Lys Pro Glu Gln Gln His Pro Leu Pro Ser Tyr Leu Glu
                85                  90                  95

Tyr Arg His Asn Ala Ser Ile Arg Val Tyr Gln Thr Asn Tyr Phe Ala
            100                 105                 110

Ser Lys Cys Ala Glu Ser Pro Asp Ser Ser Ser Lys Ser Phe Asn Ile
        115                 120                 125

Lys Leu Gly Ala Glu Met Asp Ser Leu Gly Gly Lys Cys Asp Pro Ser
    130                 135                 140

Gln Gln Lys Phe Cys Asp Gly Pro Leu Leu Pro His Thr Ala Tyr Arg
145                 150                 155                 160

Ile Ser Ile Arg Ala Phe Thr Gln Leu Phe Asp Glu Asp Leu Lys Glu
                165                 170                 175

Phe Thr Lys Pro Leu Tyr Ser Asp Thr Phe Phe Ser Met Pro Ile Thr
            180                 185                 190

Thr Glu Ser Glu Pro Leu Phe Gly Val Ile Glu Gly Val Ser Ala Gly
        195                 200                 205

Leu Phe Leu Ile Gly Met Leu Val Ala Leu Val Ala Phe Phe Ile Cys
    210                 215                 220

Arg Gln Lys Ala Ser His Ser Arg Glu Arg Pro Ser Ala Arg Leu Ser
225                 230                 235                 240

Ile Arg Arg Asp Arg Pro Leu Ser Val His Leu Asn Leu Gly Gln Lys
                245                 250                 255

Gly Asn Arg Lys Thr Ser Cys Pro Ile Lys Ile Asn Gln Phe Glu Gly
            260                 265                 270

His Phe Met Lys Leu Gln Ala Asp Ser Asn Tyr Leu Leu Ser Lys Glu
        275                 280                 285

Tyr Glu Asp Leu Lys Asp Val Gly Arg Ser Gln Ser Cys Asp Ile Ala
    290                 295                 300

Leu Leu Pro Glu Asn Arg Gly Lys Asn Arg Tyr Asn Asn Ile Leu Pro
305                 310                 315                 320

Tyr Asp Ala Ser Arg Val Lys Leu Ser Asn Val Asp Asp Asp Pro Cys
                325                 330                 335

Ser Asp Tyr Ile Asn Ala Ser Tyr Ile Pro Gly Asn Asn Phe Arg Arg
            340                 345                 350

Glu Tyr Ile Ala Thr Gln Gly Pro Leu Pro Gly Thr Lys Asp Asp Phe
        355                 360                 365

Trp Lys Met Ala Trp Glu Gln Asn Val His Asn Ile Val Met Val Thr
    370                 375                 380

Gln Cys Val Glu Lys Gly Arg Val Lys Cys Asp His Tyr Trp Pro Ala
385                 390                 395                 400

Asp Gln Asp Pro Leu Tyr Tyr Gly Asp Leu Ile Leu Gln Met Val Ser
                405                 410                 415

Glu Ser Val Leu Pro Glu Trp Thr Ile Arg Glu Phe Lys Ile Cys Ser
            420                 425                 430

Glu Glu Gln Leu Asp Ala His Arg Leu Ile Arg His Phe His Tyr Thr
        435                 440                 445

Val Trp Pro Asp His Gly Val Pro Glu Thr Thr Gln Ser Leu Ile Gln
    450                 455                 460

Phe Val Arg Thr Val Arg Asp Tyr Ile Asn Arg Ser Pro Gly Ala Gly
465                 470                 475                 480

Pro Thr Val Val His Cys Ser Ala Gly Val Gly Arg Thr Gly Thr Phe
                485                 490                 495

Val Ala Leu Asp Arg Ile Leu Gln Gln Leu Asp Phe Lys Asp Ser Val
            500                 505                 510

Asp Ile Tyr Gly Ala Val His Asp Leu Arg Leu His Arg Val His Met
        515                 520                 525

Val Gln Thr Glu Cys Gln Tyr Val Tyr Leu His Gln Cys Val Arg Asp
    530                 535                 540

Val Leu Arg Ala Lys Lys Leu Arg Asn Glu Gln Glu Asn Pro Leu Phe
545                 550                 555                 560

Pro Ile Tyr Glu Asn Val Asn Pro Glu Tyr His Arg Asp Ala Ile Tyr

565                 570                 575

Ser Arg His
            580

<210> 3
<211> 6075
<212> DNA
<213> Homo sapiens

<220>
<221> CDS
<222> (31)..(6021)

<400> 3
gtctcctctg gatcttaact actgagcgca atg ctg agc cat gga gcc ggg ttg   54
gcc ttg tgg atc aca ctg agc ctg ctg cag act gga ctg gcg gag cca   102
gag aga tgt aac ttc acc ctg gcg gag tcc aag gcc tcc agc cat tct   150
gtg tct atc cag tgg aga att ttg ggc tca ccc tgt aac ttt agc ctc   198
atc tat agc agt gac acc ctg ggg gcc gcg ttg tgc cct acc ttt cgg   246
ata gac aac acc aca tac gga tgt aac ctt caa gat tta caa gca gga   294
acc atc tat aac ttc aag att att tct ctg gat gaa gag aga act gtg   342
gtc ttg caa aca gat cct tta cct cct gct agg ttt gga gtc agt aaa   390
gag aag acg act tca acc ggc ttg cat gtt tgg tgg act cct tct tcc   438
gga aaa gtc acc tca tat gag gtg caa tta ttt gat gaa aat aac caa   486
aag ata cag ggg gtt caa att caa gaa agt act tca tgg aat gaa tac 534
act ttt ttc aat ctc act gct ggt agt aaa tac aat att gcc atc aca 582
gct gtt tct gga gga aaa cgt tct ttt tca gtt tat acc aat gga tca 630
aca gtg cca tct cca gtg aaa gat att ggt att tcc aca aaa gcc aat 678
tct ctc ctg att tcc tgg tcc cat ggt tct ggg aat gtg gaa cga tac 726
cgg ctg atg cta atg gat aaa ggg atc cta gtt cat ggc ggt gtt gtg 774
gac aaa cat gct act tcc tat gct ttt cac ggg ctg tcc cct ggc tac 822
ctc tac aac ctc act gtt atg act gag gct gca ggg ctg caa aac tac 870
agg tgg aaa cta gtc agg aca gcc ccc atg gaa gtc tca aat ctg aag 918
gtg aca aat gat ggc agt ttg acc tct cta aaa gtc aaa tgg caa aga 966
cct cct gga aat gtg gat tct tac aat atc acc ctg tct cac aaa ggg 1014
acc atc aag gaa tcc aga gta tta gca cct tgg att act gaa act cac 1062
ttt aaa gag tta gtc ccc ggt cga ctt tat caa gtt act gtc agc tgt 1110
gtc tct ggt gaa ctg tct gct cag aag atg gca gtg ggc aga aca ttt 1158
cca gac aaa gtt gca aac ctg gag gca aac aat aat ggc agg atg agg    1206
tct ctt gta gtg agc tgg tcg ccc cct gct gga gac tgg gag cag tat    1254
cgg atc cta ctc ttc aat gat tct gtg gtg ctg ctc aac atc act gtg    1302
gga aag gaa gaa aca cag tat gtc atg gat gac acg ggg ctc gta ccg    1350
gga aga cag tat gag gtg gaa gtc att gtt gag agt gga aat ttg aag    1398
aat tct gag cgt tgc caa ggc agg aca gtc ccc ctg gct gtc ctc cag    1446
ctt cgt gtc aaa cat gcc aat gaa acc tca ctg agt atc atg tgg cag    1494
acc cct gta gca gaa tgg gag aaa tac atc att tcc cta gct gac aga    1542
gac ctc tta ctg atc cac aag tca ctc tcc aaa gat gcc aaa gaa ttc    1590
act ttt act gac ctg gtg cct gga cga aaa tac atg gct aca gtc acc    1638
agt att agt gga gac tta aaa aat tcc tct tca gta aaa gga aga aca    1686
gtg cct gcc caa gtg act gac ttg cat gtg gcc aac caa gga atg acc    1734
agt agt ctg ttt act aac tgg acc cag gca caa gga gac gta gaa ttt    1782
tac caa gtc tta ctg atc cat gaa aat gtg gtc att aaa aat gaa agc 1830
atc tcc agt gag acc agc aga tac agc ttc cac tct ctc aag tcc ggc 1878
agc ctg tac tcc gtg gtg gta aca aca gtg agt gga ggg atc tct tcc 1926
cga caa gtg gtt gtg gag gga aga aca gtc cct tcc agt gtg agt gga 1974
gta acg gtg aac aat tcc ggt cgt aat gac tac ctc agc gtt tcc tgg 2022
ctc gtg gcg ccc gga gat gtg gat aac tat gag gta aca ttg tct cat 2070
gac ggc aag gtg gtt cag tcc ctt gtc att gcc aag tct gtc aga gaa 2118
tgt tcc ttc agc tcc ctc acc cca ggc cgc ctc tac acc gtg acc ata 2166
act aca agg agt ggc aag tat gaa aat cac tcc ttc agc caa gag cgg 2214
aca gtg cct gac aaa gtc cag gga gtc agt gtt agc aac tca gcc agg 2262
agt gac tat tta agg gta tcc tgg gtg cat gcc act gga gac ttt gat 2310
cac tat gaa gtc acc att aaa aac aaa aac aac ttc att caa act aaa 2358
agc att ccc aag tca gaa aac gaa tgt gta ttt gtt cag cta gtc cct 2406
gga cgg ttg tac agt gtc act gtt act aca aaa agt gga caa tat gaa 2454
gcc aat gaa caa ggg aat ggg aga aca att cca gag cct gtt aag gat    2502
cta aca ttg cgc aac agg agc act gag gac ttg cat gtg act tgg tca    2550
gga gct aat ggg gat gtc gac caa tat gag atc cag ctg ctc ttc aat    2598
gac atg aaa gta ttt cct cct ttt cac ctt gta aat acc gca acc gag    2646
tat cga ttt act tcc cta aca cca ggc cgc caa tac aaa att ctt gtc    2694
ttg acg att agc ggg gat gta cag cag tca gcc ttc att gag ggc ttc    2742
aca gtt cct agt gct gtc aaa aat att cac att tct ccc aat gga gca    2790
aca gat agc ctg acg gtg aac tgg act cct ggt ggg gga gac gtt gat    2838
tcc tac acg gtg tcg gca ttc agg cac agt caa aag gtt gac tct cag    2886
act att ccc aag cac gtc ttt gag cac acg ttc cac aga ctg gag gcc    2934
ggg gag cag tac cag atc atg att gcc tca gtc agc ggg tcc ctg aag    2982
aat cag ata aat gtg gtt ggg cgg aca gtt cca gca tct gtc caa gga    3030
gta att gca gac aat gca tac agc agt tat tcc tta ata gta agt tgg    3078
caa aaa gct gct ggt gtg gca gaa aga tat gat atc ctg ctt cta act    3126
gaa aat gga atc ctt ctg cgc aac aca tca gag cca gcc acc act aag    3174
caa cac aaa ttt gaa gat cta aca cca ggc aag aaa tac aag ata cag    3222
atc cta act gtc agt gga ggc ctc ttt agc aag gaa gcc cag act gaa    3270
ggc cga aca gtc cca gca gct gtc acc gac ctg agg atc aca gag aac    3318
tcc acc agg cac ctg tcc ttc cgc tgg acc gcc tca gag ggg gag ctc    3366
agc tgg tac aac atc ttt ttg tac aac cca gat ggg aat ctc cag gag    3414
aga gct caa gtt gac cca cta gtc cag agc ttc tct ttc cag aac ttg    3462
cta caa ggc aga atg tac aag atg gtg att gta act cac agt ggg gag    3510
ctg tct aat gag tct ttc ata ttt ggt aga aca gtc cca gcc tct gtg    3558
agt cat ctc agg ggg tcc aat cgg aac acg aca gac agc ctt tgg ttc    3606
aac tgg agt cca gcc tct ggg gac ttt gac ttt tat gag ctg att ctc    3654
tat aat ccc aat ggc aca aag aag gaa aac tgg aaa gac aag gac ctg    3702
acg gag tgg cgg ttt caa ggc ctt gtt cct gga agg aag tac gtg ctg    3750
tgg gtg gta act cac agt gga gat ctc agc aat aaa gtc aca gcg gag    3798
agc aga aca gct cca agt cct ccc agt ctt atg tca ttt gct gac att    3846
gca aac aca tcc ttg gcc atc acg tgg aaa ggg ccc cca gac tgg aca    3894
gac tac aac gac ttt gag ctg cag tgg ttg ccc aga gat gca ctt act    3942
gtc ttc aac ccc tac aac aac aga aaa tca gaa gga cgc att gtg tat    3990
ggt ctt cgt cca ggg aga tcc tat caa ttc aac gtc aag act gtc agt    4038
ggt gat tcc tgg aaa act tac agc aaa cca att ttt gga tct gtg agg    4086
aca aag cct gac aag ata caa aac ctg cat tgc cgg cct cag aac tcc    4134
acg gcc att gcc tgt tct tgg atc cct cct gat tct gac ttt gat ggt    4182
tat agt att gaa tgc cgg aaa atg gac acc caa gaa gtt gag ttt tcc    4230
aga aag ctg gag aaa gaa aaa tct ctg ctc aac atc atg atg cta gtg    4278
ccc cat aag agg tac ctg gtg tcc atc aaa gtg cag tcg gcc ggc atg    4326
acc agc gag gtg gtt gaa gac agc act atc aca atg ata gac cgc ccc    4374
cct cct cca ccc cca cac att cgt gtg aat gaa aag gat gtg cta att    4422
agc aag tct tcc atc aac ttt act gtc aac tgc agc tgg ttc agc gac    4470
acc aat gga gct gtg aaa tac ttc aca gtg gtg gtg aga gag gct gat    4518
ggc agt gat gag ctg aag cca gaa cag cag cac cct ctc cct tcc tac    4566
ctg gag tac agg cac aat gcc tcc att cgg gtg tat cag act aat tat    4614
ttt gcc agc aaa tgt gcc gaa aat cct aac agc aac tcc aag agt ttt    4662
aac att aag ctt gga gca gag atg gag agc tta ggt gga aaa cgc gat    4710
ccc act cag caa aaa ttc tgt gat gga cca ctg aag cca cac act gcc    4758
tac aga atc agc att cga gct ttt aca cag ctc ttt gat gag gac ctg    4806
aag gaa ttc aca aag cca ctc tat tca gac aca ttt ttt tct tta ccc    4854
atc act act gaa tca gag ccc ttg ttt gga gct att gaa ggt gtg agt    4902
gct ggt ctg ttt tta att ggc atg cta gtg gct gtt gtt gcc tta ttg    4950
atc tgc aga cag aaa gtg agc cat ggt cga gaa aga ccc tct gcc cgt    4998
ctg agc att cgt agg gat cga cca tta tct gtc cac tta aac ctg ggc    5046
cag aaa ggt aac cgg aaa act tct tgt cca ata aaa ata aat cag ttt    5094
gaa ggg cat ttc atg aag cta cag gct gac tcc aac tac ctt cta tcc    5142
aag gaa tac gag gag tta aaa gac gtg ggc cga aac cag tca tgt gac    5190
att gca ctc ttg ccg gag aat aga ggg aaa aat cga tac aac aat ata    5238
ttg ccc tat gat gcc acg cga gtg aag ctc tcc aat gta gat gat gat    5286
cct tgc tct gac tac atc aat gcc agc tac atc cct ggc aac aac ttc    5334
aga aga gaa tac att gtc act cag gga ccg ctt cct ggc acc aag gat    5382
gac ttc tgg aaa atg gtg tgg gaa caa aac gtt cac aac atc gtc atg    5430
gtg acc cag tgt gtt gag aag ggc cga gta aag tgt gac cat tac tgg    5478
cca gcg gac cag gat tcc ctc tac tat ggg gac ctc atc ctg cag atg    5526
ctc tca gag tcc gtc ctg cct gag tgg acc atc cgg gag ttt aag ata    5574
tgc ggt gag gaa cag ctt gat gca cac aga ctc atc cgc cac ttt cac    5622
tat acg gtg tgg cca gac cat gga gtc cca gaa acc acc cag tct ctg    5670
atc cag ttt gtg aga act gtc agg gac tac atc aac aga agc ccg ggt    5718
gct ggg ccc act gtg gtg cac tgc agt gct ggt gtg ggt agg act gga    5766
acc ttt att gca ttg gac cga atc ctc cag cag tta gac tcc aaa gac    5814
tct gtg gac att tat gga gca gtg cac gac cta aga ctt cac agg gtt    5862
cac atg gtc cag act gag tgt cag tat gtc tac cta cat cag tgt gta    5910
aga gat gtc ctc aga gca aga aag cta cgg agt gaa caa gaa aac ccc    5958
ttg ttt cca atc tat gaa aat gtg aat cca gag tat cac aga gat cca    6006
gtc tat tca agg cat tgagaatgta cctgaagagc tcctggataa aaattattca    6061
ctgtgtgatt tgtt                                                    6075



<210> 4
<211> 1997
<212> PRT
<213> Homo sapiens

<400> 4

SEQUENCE LISTING

<110> Max-Planck-Gesellschaft

<120> Interaction of vascular-endothelial protein-tyrosine
      phosphatase with the Angiopoietin receptor Tie-2

<130> 20036P EP

<140> 99 108 074.8
<141> 1999-04-23

<160> 4

<170> PatentIn Ver. 2.1

<210> 1
<211> 1839
<212> DNA
<213> Mus musculus

<220>
<221> CDS
<222> (1)..(1737)

<400> 1
aag agg tac ctg gtg tcc atc aag gtg cag tcg gcc ggc atg acc agt   48
Lys Arg Tyr Leu Val Ser Ile Lys Val Gln Ser Ala Gly Met Thr Ser
  1               5                  10                  15

gag gtg gtt gaa gat agc acc atc acc atg ata gac cgc ccg cct caa   96
Glu Val Val Glu Asp Ser Thr Ile Thr Met Ile Asp Arg Pro Pro Gln
             20                  25                  30

ccg cct cca cac atc cgt gtg aat gaa aag gat gtg cta atc agc aaa   144
Pro Pro Pro His Ile Arg Val Asn Glu Lys Asp Val Leu Ile Ser Lys
         35                  40                  45

tct tcc atc aac ttt act gtc aac tgc agc tgg ttc agc gac acc aac   192
Ser Ser Ile Asn Phe Thr Val Asn Cys Ser Trp Phe Ser Asp Thr Asn
     50                  55                  60

gga gcg gtt ggg tac ttt gct gtg gtg gtg aga gag gcc gac agc atg   240
Gly Ala Val Gly Tyr Phe Ala Val Val Val Arg Glu Ala Asp Ser Met
 65                  70                  75                  80

gat gag ttg aag cca gaa cag cag cac cct ctc cct tcc tac ctg gag   288
Asp Glu Leu Lys Pro Glu Gln Gln His Pro Leu Pro Ser Tyr Leu Glu

                85                  90                  95

tac aga cac aac gcc tcc atc cga gtc tac cag acc aat tat ttt gcc 336
Tyr Arg His Asn Ala Ser Ile Arg Val Tyr Gln Thr Asn Tyr Phe Ala
            100                 105                 110

agc aaa tgt gct gaa agt ccc gac agc agt tct aaa agt ttc aac att 384
Ser Lys Cys Ala Glu Ser Pro Asp Ser Ser Ser Lys Ser Phe Asn Ile
        115                 120                 125

aag ctt gga gca gag atg gac agc ctc ggt ggc aaa tgt gat ccc agt 432
Lys Leu Gly Ala Glu Met Asp Ser Leu Gly Gly Lys Cys Asp Pro Ser
    130                 135                 140

cag cag aaa ttc tgt gat gga ccg ctg ttg cca cac acc gcc tac aga 480
Gln Gln Lys Phe Cys Asp Gly Pro Leu Leu Pro His Thr Ala Tyr Arg
145                 150                 155                 160

atc agc atc cgg gct ttt aca cag cta ttt gac gag gac ttg aaa gag 528
Ile Ser Ile Arg Ala Phe Thr Gln Leu Phe Asp Glu Asp Leu Lys Glu
                165                 170                 175

ttc acc aaa cct ctc tac tcg gat acg ttc ttc tct atg ccc atc acc 576
Phe Thr Lys Pro Leu Tyr Ser Asp Thr Phe Phe Ser Met Pro Ile Thr
            180                 185                 190

aca gag tca gag ccc ttg ttt gga gtt att gaa ggt gtg agt gct ggc 624
Thr Glu Ser Glu Pro Leu Phe Gly Val Ile Glu Gly Val Ser Ala Gly
        195                 200                 205

ctg ttt cta att ggc atg ctg gtg gcc ctt gtt gcc ttc ttc atc tgc 672
Leu Phe Leu Ile Gly Met Leu Val Ala Leu Val Ala Phe Phe Ile Cys
    210                 215                 220

aga cag aaa gct agc cac agc agg gaa agg cca tct gcc cgg ctc agc 720
Arg Gln Lys Ala Ser His Ser Arg Glu Arg Pro Ser Ala Arg Leu Ser
225                 230                 235                 240

att cgt agg gac cgg cct ttg tct gtc cat ctg aat ctg ggc cag aaa 768
Ile Arg Arg Asp Arg Pro Leu Ser Val His Leu Asn Leu Gly Gln Lys
                245                 250                 255

ggc aac cgg aaa act tct tgc ccc ata aag atc aat cag ttt gaa ggg 816
Gly Asn Arg Lys Thr Ser Cys Pro Ile Lys Ile Asn Gln Phe Glu Gly
            260                 265                 270

cat ttc atg aag ctg cag gca gac tcc aac tac ctt cta tcc aag gaa 864
His Phe Met Lys Leu Gln Ala Asp Ser Asn Tyr Leu Leu Ser Lys Glu

275                 280                 285

tat gag gac tta aaa gac gtg ggt aga agc cag tca tgt gac att gcc    912
Tyr Glu Asp Leu Lys Asp Val Gly Arg Ser Gln Ser Cys Asp Ile Ala
    290                 295                 300

ctc ttg cct gag aat cga ggg aaa aat cga tac aac aac ata ttg cct    960
Leu Leu Pro Glu Asn Arg Gly Lys Asn Arg Tyr Asn Asn Ile Leu Pro
305                 310                 315                 320

tat gat gcc tca aga gtg aag ctc tcg aat gtc gat gac gac cct tgc    1008
Tyr Asp Ala Ser Arg Val Lys Leu Ser Asn Val Asp Asp Asp Pro Cys
                325                 330                 335

tct gac tac atc aac gcc agc tac atc ccc ggt aac aac ttc aga cga    1056
Ser Asp Tyr Ile Asn Ala Ser Tyr Ile Pro Gly Asn Asn Phe Arg Arg
            340                 345                 350

gaa tac atc gcc act cag gga ccg ctt cca ggc acc aag gat gac ttc    1104
Glu Tyr Ile Ala Thr Gln Gly Pro Leu Pro Gly Thr Lys Asp Asp Phe
        355                 360                 365

tgg aag atg gcg tgg gag cag aac gtt cac aac atc gtc atg gtg acc    1152
Trp Lys Met Ala Trp Glu Gln Asn Val His Asn Ile Val Met Val Thr
    370                 375                 380

cag tgt gtt gaa aag ggc cga gtg aag tgt gac cat tac tgg cca gca    1200
Gln Cys Val Glu Lys Gly Arg Val Lys Cys Asp His Tyr Trp Pro Ala
385                 390                 395                 400

gac cag gac ccc ctc tac tac ggt gat ctc atc cta cag atg gtc tcg    1248
Asp Gln Asp Pro Leu Tyr Tyr Gly Asp Leu Ile Leu Gln Met Val Ser
                405                 410                 415

gag tcc gtg ctc ccc gag tgg acc atc agg gag ttt aag ata tgc agt    1296
Glu Ser Val Leu Pro Glu Trp Thr Ile Arg Glu Phe Lys Ile Cys Ser
            420                 425                 430

gaa gaa cag ttg gat gca cac aga ctc atc cgt cac ttt cac tac acg    1344
Glu Glu Gln Leu Asp Ala His Arg Leu Ile Arg His Phe His Tyr Thr
        435                 440                 445

gtg tgg cca gac cat ggg gtc cca gag acc acc cag tcc ctg atc caa    1392
Val Trp Pro Asp His Gly Val Pro Glu Thr Thr Gln Ser Leu Ile Gln
    450                 455                 460

ttt gtg agg aca gtc agg gac tac atc aac aga agc ccc ggg gct ggg    1440
Phe Val Arg Thr Val Arg Asp Tyr Ile Asn Arg Ser Pro Gly Ala Gly

465                 470                 475                 480

ccc acc gta gtg cac tgc agc gct ggt gtg ggc aga aca ggg acg ttc    1488
Pro Thr Val Val His Cys Ser Ala Gly Val Gly Arg Thr Gly Thr Phe
                485                 490                 495

gtt gcc ctg gac cgg atc ctc cag cag ttg gac ttt aag gac tcc gtg    1536
Val Ala Leu Asp Arg Ile Leu Gln Gln Leu Asp Phe Lys Asp Ser Val
            500                 505                 510

gac att tat ggg gca gtg cat gac cta aga ctc cac agg gtt cac atg    1584
Asp Ile Tyr Gly Ala Val His Asp Leu Arg Leu His Arg Val His Met
        515                 520                 525

gtc cag acc gag tgt caa tat gtg tat ctg cat cag tgt gta aga gac    1632
Val Gln Thr Glu Cys Gln Tyr Val Tyr Leu His Gln Cys Val Arg Asp
    530                 535                 540

gtc ctc aga gca aag aaa ctg cgg aac gag caa gag aac ccc ctg ttt    1680
Val Leu Arg Ala Lys Lys Leu Arg Asn Glu Gln Glu Asn Pro Leu Phe
545                 550                 555                 560

ccg att tat gag aat gtg aat cca gag tat cac aga gat gca atc tac    1728
Pro Ile Tyr Glu Asn Val Asn Pro Glu Tyr His Arg Asp Ala Ile Tyr
                565                 570                 575

tcg aga cat taagaattca cctgaagatc ccctggataa aagcgtttca            1777
Ser Arg His

ctgtgtgact ttaaaaaaaa aaaaaaaaaa aactcgaggg ggggcccgta cccaatcnna 1837

aa                                                                 1839

<210> 2
<211> 579
<212> PRT
<213> Mus musculus

<400> 2
Lys Arg Tyr Leu Val Ser Ile Lys Val Gln Ser Ala Gly Met Thr Ser
  1               5                  10                  15

Glu Val Val Glu Asp Ser Thr Ile Thr Met Ile Asp Arg Pro Pro Gln
             20                  25                  30

Pro Pro Pro His Ile Arg Val Asn Glu Lys Asp Val Leu Ile Ser Lys
         35                  40                  45

Ser Ser Ile Asn Phe Thr Val Asn Cys Ser Trp Phe Ser Asp Thr Asn
    50                  55                  60

Gly Ala Val Gly Tyr Phe Ala Val Val Val Arg Glu Ala Asp Ser Met
65                  70                  75                  80

Asp Glu Leu Lys Pro Glu Gln Gln His Pro Leu Pro Ser Tyr Leu Glu
                85                  90                  95

Tyr Arg His Asn Ala Ser Ile Arg Val Tyr Gln Thr Asn Tyr Phe Ala
            100                 105                 110

Ser Lys Cys Ala Glu Ser Pro Asp Ser Ser Ser Lys Ser Phe Asn Ile
        115                 120                 125

Lys Leu Gly Ala Glu Met Asp Ser Leu Gly Gly Lys Cys Asp Pro Ser
    130                 135                 140

Gln Gln Lys Phe Cys Asp Gly Pro Leu Leu Pro His Thr Ala Tyr Arg
145                 150                 155                 160

Ile Ser Ile Arg Ala Phe Thr Gln Leu Phe Asp Glu Asp Leu Lys Glu
                165                 170                 175

Phe Thr Lys Pro Leu Tyr Ser Asp Thr Phe Phe Ser Met Pro Ile Thr
            180                 185                 190

Thr Glu Ser Glu Pro Leu Phe Gly Val Ile Glu Gly Val Ser Ala Gly
        195                 200                 205

Leu Phe Leu Ile Gly Met Leu Val Ala Leu Val Ala Phe Phe Ile Cys
    210                 215                 220

Arg Gln Lys Ala Ser His Ser Arg Glu Arg Pro Ser Ala Arg Leu Ser
225                 230                 235                 240

Ile Arg Arg Asp Arg Pro Leu Ser Val His Leu Asn Leu Gly Gln Lys
                245                 250                 255

Gly Asn Arg Lys Thr Ser Cys Pro Ile Lys Ile Asn Gln Phe Glu Gly
            260                 265                 270

His Phe Met Lys Leu Gln Ala Asp Ser Asn Tyr Leu Leu Ser Lys Glu
        275                 280                 285

Tyr Glu Asp Leu Lys Asp Val Gly Arg Ser Gln Ser Cys Asp Ile Ala
    290                 295                 300

Leu Leu Pro Glu Asn Arg Gly Lys Asn Arg Tyr Asn Asn Ile Leu Pro
305                 310                 315                 320

Tyr Asp Ala Ser Arg Val Lys Leu Ser Asn Val Asp Asp Asp Pro Cys
                325                 330                 335

Ser Asp Tyr Ile Asn Ala Ser Tyr Ile Pro Gly Asn Asn Phe Arg Arg
            340                 345                 350

Glu Tyr Ile Ala Thr Gln Gly Pro Leu Pro Gly Thr Lys Asp Asp Phe
        355                 360                 365

Trp Lys Met Ala Trp Glu Gln Asn Val His Asn Ile Val Met Val Thr
    370                 375                 380

Gln Cys Val Glu Lys Gly Arg Val Lys Cys Asp His Tyr Trp Pro Ala
385                 390                 395                 400

Asp Gln Asp Pro Leu Tyr Tyr Gly Asp Leu Ile Leu Gln Met Val Ser
                405                 410                 415

Glu Ser Val Leu Pro Glu Trp Thr Ile Arg Glu Phe Lys Ile Cys Ser
            420                 425                 430

Glu Glu Gln Leu Asp Ala His Arg Leu Ile Arg His Phe His Tyr Thr
        435                 440                 445

Val Trp Pro Asp His Gly Val Pro Glu Thr Thr Gln Ser Leu Ile Gln
    450                 455                 460

Phe Val Arg Thr Val Arg Asp Tyr Ile Asn Arg Ser Pro Gly Ala Gly
465                 470                 475                 480

Pro Thr Val Val His Cys Ser Ala Gly Val Gly Arg Thr Gly Thr Phe
                485                 490                 495

Val Ala Leu Asp Arg Ile Leu Gln Gln Leu Asp Phe Lys Asp Ser Val
            500                 505                 510

Asp Ile Tyr Gly Ala Val His Asp Leu Arg Leu His Arg Val His Met
        515                 520                 525

Val Gln Thr Glu Cys Gln Tyr Val Tyr Leu His Gln Cys Val Arg Asp
    530                 535                 540

Val Leu Arg Ala Lys Lys Leu Arg Asn Glu Gln Glu Asn Pro Leu Phe
545                 550                 555                 560

Pro Ile Tyr Glu Asn Val Asn Pro Glu Tyr His Arg Asp Ala Ile Tyr
                565                 570                 575

Ser Arg His

<210> 3
<211> 6075
<212> DNA
<213> Homo sapiens

<220>
<221> CDS
<222> (31)..(6021)

<400> 3
gtctcctctg gatcttaact actgagcgca atg ctg agc cat gga gcc ggg ttg   54
gcc ttg tgg atc aca ctg agc ctg ctg cag act gga ctg gcg gag cca   102
gag aga tgt aac ttc acc ctg gcg gag tcc aag gcc tcc agc cat tct   150
gtg tct atc cag tgg aga att ttg ggc tca ccc tgt aac ttt agc ctc   198
atc tat agc agt gac acc ctg ggg gcc gcg ttg tgc cct acc ttt cgg   246
ata gac aac acc aca tac gga tgt aac ctt caa gat tta caa gca gga   294
acc atc tat aac ttc aag att att tct ctg gat gaa gag aga act gtg   342
gtc ttg caa aca gat cct tta cct cct gct agg ttt gga gtc agt aaa   390
gag aag acg act tca acc ggc ttg cat gtt tgg tgg act cct tct tcc    438
gga aaa gtc acc tca tat gag gtg caa tta ttt gat gaa aat aac caa    486
aag ata cag ggg gtt caa att caa gaa agt act tca tgg aat gaa tac    534
act ttt ttc aat ctc act gct ggt agt aaa tac aat att gcc atc aca    582
gct gtt tct gga gga aaa cgt tct ttt tca gtt tat acc aat gga tca    630
aca gtg cca tct cca gtg aaa gat att ggt att tcc aca aaa gcc aat    678
tct ctc ctg att tcc tgg tcc cat ggt tct ggg aat gtg gaa cga tac    726
cgg ctg atg cta atg gat aaa ggg atc cta gtt cat ggc ggt gtt gtg    774
gac aaa cat gct act tcc tat gct ttt cac ggg ctg tcc cct ggc tac    822
ctc tac aac ctc act gtt atg act gag gct gca ggg ctg caa aac tac    870
agg tgg aaa cta gtc agg aca gcc ccc atg gaa gtc tca aat ctg aag    918
gtg aca aat gat ggc agt ttg acc tct cta aaa gtc aaa tgg caa aga    966
cct cct gga aat gtg gat tct tac aat atc acc ctg tct cac aaa ggg 1014
acc atc aag gaa tcc aga gta tta gca cct tgg att act gaa act cac 1062
ttt aaa gag tta gtc ccc ggt cga ctt tat caa gtt act gtc agc tgt 1110
gtc tct ggt gaa ctg tct gct cag aag atg gca gtg ggc aga aca ttt 1158
cca gac aaa gtt gca aac ctg gag gca aac aat aat ggc agg atg agg 1206
tct ctt gta gtg agc tgg tcg ccc cct gct gga gac tgg gag cag tat 1254
cgg atc cta ctc ttc aat gat tct gtg gtg ctg ctc aac atc act gtg 1302
gga aag gaa gaa aca cag tat gtc atg gat gac acg ggg ctc gta ccg 1350
gga aga cag tat gag gtg gaa gtc att gtt gag agt gga aat ttg aag 1398
aat tct gag cgt tgc caa ggc agg aca gtc ccc ctg gct gtc ctc cag 1446
ctt cgt gtc aaa cat gcc aat gaa acc tca ctg agt atc atg tgg cag 1494
acc cct gta gca gaa tgg gag aaa tac atc att tcc cta gct gac aga 1542
gac ctc tta ctg atc cac aag tca ctc tcc aaa gat gcc aaa gaa ttc 1590
act ttt act gac ctg gtg cct gga cga aaa tac atg gct aca gtc acc 1638
agt att agt gga gac tta aaa aat tcc tct tca gta aaa gga aga aca 1686
gtg cct gcc caa gtg act gac ttg cat gtg gcc aac caa gga atg acc 1734
agt agt ctg ttt act aac tgg acc cag gca caa gga gac gta gaa ttt 1782
tac caa gtc tta ctg atc cat gaa aat gtg gtc att aaa aat gaa agc 1830
atc tcc agt gag acc agc aga tac agc ttc cac tct ctc aag tcc ggc 1878
agc ctg tac tcc gtg gtg gta aca aca gtg agt gga ggg atc tct tcc 1926
cga caa gtg gtt gtg gag gga aga aca gtc cct tcc agt gtg agt gga 1974
gta acg gtg aac aat tcc ggt cgt aat gac tac ctc agc gtt tcc tgg 2022
ctc gtg gcg ccc gga gat gtg gat aac tat gag gta aca ttg tct cat 2070
gac ggc aag gtg gtt cag tcc ctt gtc att gcc aag tct gtc aga gaa 2118
tgt tcc ttc agc tcc ctc acc cca ggc cgc ctc tac acc gtg acc ata 2166
act aca agg agt ggc aag tat gaa aat cac tcc ttc agc caa gag cgg 2214
aca gtg cct gac aaa gtc cag gga gtc agt gtt agc aac tca gcc agg 2262
agt gac tat tta agg gta tcc tgg gtg cat gcc act gga gac ttt gat 2310
cac tat gaa gtc acc att aaa aac aaa aac aac ttc att caa act aaa 2358
agc att ccc aag tca gaa aac gaa tgt gta ttt gtt cag cta gtc cct 2406
gga cgg ttg tac agt gtc act gtt act aca aaa agt gga caa tat gaa 2454
gcc aat gaa caa ggg aat ggg aga aca att cca gag cct gtt aag gat 2502
cta aca ttg cgc aac agg agc act gag gac ttg cat gtg act tgg tca 2550
gga gct aat ggg gat gtc gac caa tat gag atc cag ctg ctc ttc aat 2598
gac atg aaa gta ttt cct cct ttt cac ctt gta aat acc gca acc gag 2646
tat cga ttt act tcc cta aca cca ggc cgc caa tac aaa att ctt gtc 2694
ttg acg att agc ggg gat gta cag cag tca gcc ttc att gag ggc ttc     2742
aca gtt cct agt gct gtc aaa aat att cac att tct ccc aat gga gca     2790
aca gat agc ctg acg gtg aac tgg act cct ggt ggg gga gac gtt gat     2838
tcc tac acg gtg tcg gca ttc agg cac agt caa aag gtt gac tct cag     2886
act att ccc aag cac gtc ttt gag cac acg ttc cac aga ctg gag gcc     2934
ggg gag cag tac cag atc atg att gcc tca gtc agc ggg tcc ctg aag     2982
aat cag ata aat gtg gtt ggg cgg aca gtt cca gca tct gtc caa gga     3030
gta att gca gac aat gca tac agc agt tat tcc tta ata gta agt tgg     3078
caa aaa gct gct ggt gtg gca gaa aga tat gat atc ctg ctt cta act     3126
gaa aat gga atc ctt ctg cgc aac aca tca gag cca gcc acc act aag     3174
caa cac aaa ttt gaa gat cta aca cca ggc aag aaa tac aag ata cag     3222
atc cta act gtc agt gga ggc ctc ttt agc aag gaa gcc cag act gaa     3270
ggc cga aca gtc cca gca gct gtc acc gac ctg agg atc aca gag aac     3318
tcc acc agg cac ctg tcc ttc cgc tgg acc gcc tca gag ggg gag ctc     3366
agc tgg tac aac atc ttt ttg tac aac cca gat ggg aat ctc cag gag     3414
aga gct caa gtt gac cca cta gtc cag agc ttc tct ttc cag aac ttg     3462
cta caa ggc aga atg tac aag atg gtg att gta act cac agt ggg gag     3510
ctg tct aat gag tct ttc ata ttt ggt aga aca gtc cca gcc tct gtg     3558
agt cat ctc agg ggg tcc aat cgg aac acg aca gac agc ctt tgg ttc     3606
aac tgg agt cca gcc tct ggg gac ttt gac ttt tat gag ctg att ctc     3654
tat aat ccc aat ggc aca aag aag gaa aac tgg aaa gac aag gac ctg     3702
acg gag tgg cgg ttt caa ggc ctt gtt cct gga agg aag tac gtg ctg     3750
tgg gtg gta act cac agt gga gat ctc agc aat aaa gtc aca gcg gag     3798
agc aga aca gct cca agt cct ccc agt ctt atg tca ttt gct gac att     3846
gca aac aca tcc ttg gcc atc acg tgg aaa ggg ccc cca gac tgg aca 3894
gac tac aac gac ttt gag ctg cag tgg ttg ccc aga gat gca ctt act 3942
gtc ttc aac ccc tac aac aac aga aaa tca gaa gga cgc att gtg tat 3990
ggt ctt cgt cca ggg aga tcc tat caa ttc aac gtc aag act gtc agt 4038
ggt gat tcc tgg aaa act tac agc aaa cca att ttt gga tct gtg agg 4086
aca aag cct gac aag ata caa aac ctg cat tgc cgg cct cag aac tcc 4134
acg gcc att gcc tgt tct tgg atc cct cct gat tct gac ttt gat ggt 4182
tat agt att gaa tgc cgg aaa atg gac acc caa gaa gtt gag ttt tcc 4230
aga aag ctg gag aaa gaa aaa tct ctg ctc aac atc atg atg cta gtg 4278
ccc cat aag agg tac ctg gtg tcc atc aaa gtg cag tcg gcc ggc atg 4326
acc agc gag gtg gtt gaa gac agc act atc aca atg ata gac cgc ccc 4374
cct cct cca ccc cca cac att cgt gtg aat gaa aag gat gtg cta att 4422
agc aag tct tcc atc aac ttt act gtc aac tgc agc tgg ttc agc gac    4470
acc aat gga gct gtg aaa tac ttc aca gtg gtg gtg aga gag gct gat    4518
ggc agt gat gag ctg aag cca gaa cag cag cac cct ctc cct tcc tac    4566
ctg gag tac agg cac aat gcc tcc att cgg gtg tat cag act aat tat    4614
ttt gcc agc aaa tgt gcc gaa aat cct aac agc aac tcc aag agt ttt    4662
aac att aag ctt gga gca gag atg gag agc tta ggt gga aaa cgc gat    4710
ccc act cag caa aaa ttc tgt gat gga cca ctg aag cca cac act gcc    4758
tac aga atc agc att cga gct ttt aca cag ctc ttt gat gag gac ctg    4806
aag gaa ttc aca aag cca ctc tat tca gac aca ttt ttt tct tta ccc    4854
atc act act gaa tca gag ccc ttg ttt gga gct att gaa ggt gtg agt    4902
gct ggt ctg ttt tta att ggc atg cta gtg gct gtt gtt gcc tta ttg    4950
atc tgc aga cag aaa gtg agc cat ggt cga gaa aga ccc tct gcc cgt    4998
ctg agc att cgt agg gat cga cca tta tct gtc cac tta aac ctg ggc    5046
cag aaa ggt aac cgg aaa act tct tgt cca ata aaa ata aat cag ttt    5094
gaa ggg cat ttc atg aag cta cag gct gac tcc aac tac ctt cta tcc    5142
aag gaa tac gag gag tta aaa gac gtg ggc cga aac cag tca tgt gac    5190
att gca ctc ttg ccg gag aat aga ggg aaa aat cga tac aac aat ata    5238
ttg ccc tat gat gcc acg cga gtg aag ctc tcc aat gta gat gat gat    5286
cct tgc tct gac tac atc aat gcc agc tac atc cct ggc aac aac ttc    5334
aga aga gaa tac att gtc act cag gga ccg ctt cct ggc acc aag gat    5382
gac ttc tgg aaa atg gtg tgg gaa caa aac gtt cac aac atc gtc atg    5430
gtg acc cag tgt gtt gag aag ggc cga gta aag tgt gac cat tac tgg    5478
cca gcg gac cag gat tcc ctc tac tat ggg gac ctc atc ctg cag atg    5526
ctc tca gag tcc gtc ctg cct gag tgg acc atc cgg gag ttt aag ata    5574
tgc ggt gag gaa cag ctt gat gca cac aga ctc atc cgc cac ttt cac 5622
tat acg gtg tgg cca gac cat gga gtc cca gaa acc acc cag tct ctg 5670
atc cag ttt gtg aga act gtc agg gac tac atc aac aga agc ccg ggt 5718
gct ggg ccc act gtg gtg cac tgc agt gct ggt gtg ggt agg act gga 5766
acc ttt att gca ttg gac cga atc ctc cag cag tta gac tcc aaa gac 5814
tct gtg gac att tat gga gca gtg cac gac cta aga ctt cac agg gtt 5862
cac atg gtc cag act gag tgt cag tat gtc tac cta cat cag tgt gta 5910
aga gat gtc ctc aga gca aga aag cta cgg agt gaa caa gaa aac ccc 5958
ttg ttt cca atc tat gaa aat gtg aat cca gag tat cac aga gat cca 6006
gtc tat tca agg cat tgagaatgta cctgaagagc tcctggataa aaattattca 6061
ctgtgtgatt tgtt 6075

<210> 4
<211> 1997

<212> PRT
<213> Homo sapiens

<400> 4